# EUROPEAN PATENT APPLICATION

(11) **EP 1 340 458 A1**
(43) Date of publication of application: **03.09.2003**
(21) Application number: 03001229.8
(22) Date of filing: 20.01.2003
(51) Int. Cl.: A61B 5/15

(54) **Shieldable fluid collection set**

(30) Priority: 28.02.2002 US 361447 P
(71) Applicant: Becton Dickinson and Company, Franklin Lakes, New Jersey 07417-1880 (US)
(72) Inventor: Swenson, Kirk D., North Caldwell, New Jersey 07006 (US); Newby, Mark, Tuxedo, New York 10987 (US); Crawford, Jamieson William Maclean, New York 10025 (US)
(74) Representative: Müller-Boré & Partner Patentanwälte

(57) **Abstract**

A fluid collection or infusion set is provided with a needle assembly that is slidably disposed in a generally tubular shield. The needle assembly includes two opposed flexible wings that are slidably receivable in at least one slot of the shield. The wings can be rotated from a coplanar disposition into a position where the wings are in face-to-face engagement with one another. A flexible bridge may extend between the wings to facilitate manipulation of the needle assembly. The shield may be tapered to wider dimensions closer to the proximal end of the shield. The wings may include lugs that engage external surfaces of the shield and cause the wings to stretch as the shield is moved distally over the needle assembly. The lugs then snap into recesses at the proximal end of the shield or preventing re-exposure of the needle. Alternatively, the shield may include a locking clip that can be rotated into a position for covering portions of the slot in the shield to prevent re-exposure of the needle.

## Description

### RELATED APPLICATION

This application claims priority on U.S. Provisional Patent Appl. No. 60/361,447 which was filed on February 28, 2002.

### FIELD OF THE INVENTION

The invention relates to a medical apparatus with a retractable needle for fluid collection or infusion.

### BACKGROUND OF THE INVENTION

Needle cannulas are employed for collecting blood or other bodily fluids from a patient or for infusing blood, drugs or other liquids into a patient. The needle cannula typically is mounted to a plastic hub, which in turn is mounted to a device for collecting or infusing the liquid. One such device includes a length of flexible plastic tubing with a distal end connected to the needle hub and a proximal end connected to a plastic fitting. The fitting at the proximal end of the plastic tubing can take many forms depending on the intended use of the device. Devices of this type often are referred to as blood collection sets, fluid collection sets or intravenous infusion sets, depending upon the intended use of the device.

The above-described medical devices often include a pair of flexible plastic wings mounted to or near the needle hub. The wings can be folded into face-to-face engagement with one another, and hence define a convenient handle for gripping and manipulating the needle cannula. The wings also can be rotated away from one another and can be taped into face-to-face contact with the skin of the patient.

Accidental sticks with a needle cannula can be painful and can transmit disease. As a result, most needle assemblies are employed with rigid means for enclosing the needle cannula both prior to use and after use. Protection prior to use typically is achieved by a rigid plastic tube that has a proximal end frictionally mounted to the needle hub and a distal end that extends beyond the distal end of the needle cannula. The plastic tube is removed and discarded immediately prior to use of the needle cannula. Protection after use of the needle cannula typically is achieved by a tubular shield that can be telescoped relative to the needle hub and needle cannula from a proximal position where the needle is enclosed to a distal position where the needle cannula is safely within the tubular shield. Shields of this type typically include means for releasably holding the shield in its proximal position and for holding the shield more securely in its distal position. The retention of the shield in its distal position should prevent any accidental re-exposure of the used needle cannula and preferably should prevent or substantially complicate an intentional attempt to reuse the needle cannula.

### SUMMARY OF THE INVENTION

The invention is directed to a fluid collection or infusion set that comprises a length of flexible plastic tubing with opposite proximal and distal ends and a passage extending between the ends. A fitting is securely connected to the proximal end of the flexible plastic tubing.

The fluid collection or infusion set further comprises a needle assembly. The needle assembly includes a needle hub with a proximal end, a distal end and a passage extending between the ends. Portions of the passage adjacent the proximal end of the hub are securely engaged with the distal end of the flexible plastic tubing.

The needle assembly further includes a needle cannula having opposite proximal and distal ends and a lumen extending between the ends. Thus, the lumen through the needle cannula communicates with the passage through the needle hub and with the passage through the flexible tubing. The needle assembly may further include a needle protector removably mounted over the needle cannula and extending sufficiently to cover the distal end of the needle cannula.

Two opposed flexible wings project transversely from the needle hub. The wings can be folded into substantially face-to-face relationship with one another to facilitate gripping of the needle assembly between a thumb and forefinger. Thus, the folded wings effectively function as a handle to facilitate manipulation of the needle assembly. The wings also can be folded into a substantially coplanar disposition for taping the needle assembly onto the skin of a patient. Additionally, the wings may include a flexible bridge extending from one wing to the other at locations spaced from the hinged connection of the wings to the needle hub. The bridge can be collapsed between the wings when the wings are folded into face-to-face engagement with one another. However, the bridge projects from the wings when the wings are in their coplanar disposition, and hence defines an actuator for facilitating movement of the needle assembly when the wings are in their coplanar disposition. Each of the wings may be formed with a lug projecting from the plane defined by each wing.

The fluid collection or infusion set further comprises a generally tubular shield telescoped over the needle assembly. The shield includes opposite proximal and distal ends and a passage extending between the ends. The needle hub is dimensioned to move slidably in the passage from a distal position where the needle cannula is exposed to a proximal position where the needle cannula is safely disposed within the shield. The shield includes two opposed slots that extend from a location at or near the distal end to a location at or near the proximal end. The slots are dimensioned to slidably accommodate the wings so that the needle assembly and the wings can be moved from the distal position to the proximal position relative to the shields. Proximal portions of the slots may be enlarged to define a recess into which the wings will be received when the needle assembly reaches the proximal position. Thus, the wings can be trapped in the recesses for complicating or preventing movement of the needle assembly back toward the distal position.

The external width of the shield may vary gradually from a minor dimension at the distal end of the slots to a major dimension at the proximal ends of the slots. More particularly, the external width of the shield at the distal end preferably is less than or equal to the distance between the lugs on the wings. However, the external width of the shield at the proximal end of the slots preferably exceeds the distance between the lugs. As a result, the initial proximal movement of the needle assembly relative to the shield can be carried out easily without interference between the lugs and the shield. Further movement, however, causes the lugs to engage the exterior of the shield. Hence, the wings stretch slightly away from one another as the needle assembly moves further in the proximal direction. The lugs align with the recesses at the proximal ends of the slots when the needle assembly reaches its proximal position. Thus, the wings return resiliently to an unstretched condition and the lugs move into the recess at the proximal end of the slot for securely trapping the needle assembly in the proximal position. A return distal movement of the needle assembly would require a very complicated stretching of the wings by hand while simultaneously displacing the wings and needle assembly distally relative to the shield.

In an alternate embodiment, the shield may comprise only a single slot and may have a cylindrical outer surface. The shield may further include a rotatable split clip mounted over the cylindrical outer surface of the shield. The clip may initially be in a rotational orientation with the split in the clip aligned with the slot in the shield. Shielding of this embodiment is achieved by rotating the wings into face-to-face engagement with one another and sliding the shield distally over the needle assembly so that the folded wings slide into and through the single slot of the shield and the slit in the rotatable clip. The clip may be rotated relative to the shield after the wings have moved proximally beyond the rotatable clip. Thus, the clip prevents a return distal movement of the needle assembly.

### BRIEF DESCRIPTION OF THE DRAWINGS

FIG. 1 is a perspective view of a fluid collection or infusion set in accordance with a first embodiment of the invention.

FIG. 2 is an end elevational view of the fluid collection or infusion set of FIG. 1.

FIG. 3 is an end elevational view similar to FIG. 2, but showing the wings in a folded condition.

FIG. 4 is a perspective view of a second embodiment of a fluid collection or infusion set in accordance with the subject invention with the needle assembly in a ready-to-use position.

FIG. 5 is a longitudinal cross-sectional view of the fluid collection set of FIG. 4 prior to shielding.

FIG. 6 is a cross-sectional view taken along line 6-6 in FIG. 5.

FIG. 7 is a cross-sectional view similar to FIG. 5, but showing the needle assembly in a partly shielded condition.

FIG. 8 is a cross-sectional view similar to FIGS. 5 and 7, but showing the needle assembly in a fully shielded condition.

FIG. 9 is a perspective view of a third embodiment of the fluid collection or infusion set in accordance with the subject invention with the needle assembly in a ready-to-use condition.

FIG. 10 is a perspective view of the fluid collection or infusion set of FIG. 9 with the needle assembly in a fully shielded condition.

### DETAILED DESCRIPTION OF THE INVENTION

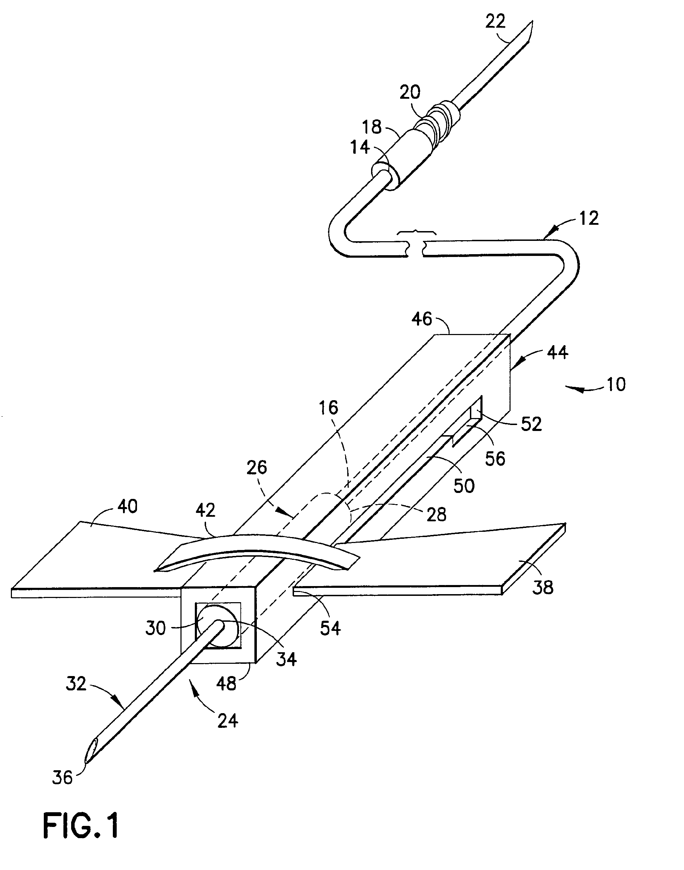

A fluid collection set in accordance with a first embodiment of the invention is identified generally by the numeral **10** in FIGS. 1-3. Fluid collection set **10** includes a length of flexible plastic tubing **12** with a proximal end **14** and a distal end **16**. Proximal end **14** of flexible tubing **12** is connected to a plastic fitting **18**. Fitting **18** can take many different forms. However, fitting **18** shown in FIG. 1 includes an array of external threads **20** and a proximal needle cannula **22** to enable fitting **18** to be connected with a holder (not shown) for receiving an evacuated blood collection tube.

Fluid collection set **10** further includes a needle assembly **24**. Needle assembly **24** includes a plastic needle hub **26** with a proximal end **28**, a distal end **30** and a passage (not shown) extending between the ends. Distal end **16** of plastic tubing **20** is connected to the passage of hub **26** adjacent proximal end **28** so that the passage through needle hub **26** communicates with the passage through flexible tubing **12**. Needle assembly **24** further includes a needle cannula **32** with a proximal end **34**, a distal end **36** and a lumen extending between the ends. Proximal end **34** of needle cannula **32** is securely mounted in the passage of needle hub **26** adjacent distal end **30** of needle hub **26**.

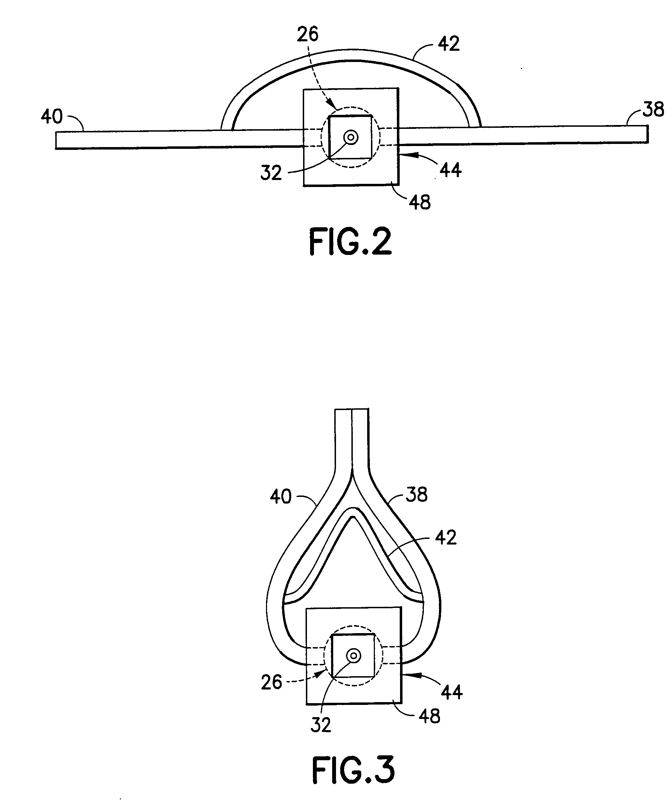

Needle assembly **24** further includes flexible wings **38** and **40** extending transversely from needle hub **26**. Wings **38** and **40** can be rotated relative to needle hub **26** from the coplanar orientation shown in FIGS. 1 and 2 into the condition shown in FIG. 3 where at least upper portions of wings **38** and **40** are in substantially face-to-face engagement with one another.

Needle assembly **24** is further characterized by a collapsible bridge **42** extending between and connecting central positions on wings **38** and **40**. Bridge **42** defines a generally arch shape when wings **38** and **40** are in the coplanar orientation shown in FIGS. 1 and 2. However, bridge **42** collapses upon itself when wings **38** and **40** are rotated toward one another and into the generally face-to-face disposition shown in FIG. 3.

Fluid collection or infusion set **10** further includes a generally tubular shield **44** with opposite proximal and distal ends **46** and **48** and a passage extending between the ends. The passage of tubular shield **44** is dimensioned to slidably accommodate needle hub **26** so that needle assembly **24** can be moved from a distal position shown in FIG. 1 where needle cannula **32** projects distally from shield **44** to a proximal position where needle cannula **32** is enclosed within shield **44**.

Shield **44** is characterized by longitudinal slots **50**. Each slot **50** includes a proximal end **52** near proximal end **46** of shield **44** and a distal end **54** near distal end **48** of shield **44**. Portions of slots **50** extending proximally from distal end **54** are dimensioned to slidably engage portions of wings **38** and **40** inwardly from bridge **42**. However, portions of slots **50** adjacent proximal end **52** define a recess **56** for locked engagement with wings **38** and **40**.

Fluid collection or infusion set **10** initially is in the FIG. 1 orientation with needle cannula **32** projecting distally beyond shield **44**. Wings **38** and **40** can be in the coplanar disposition shown in FIGS. 1 and 2, with bridge **42** extending between wings **38** and **40** and over portions of shield **44**. Thus, bridge **42** defines an engagement surface that can facilitate one-handed manipulation of needle assembly **24**. More particularly, proximal portions of shield **44** can be engaged between a thumb and forefinger, while an index finger may be disposed against bridge **42** to facilitate guiding and manipulation of needle assembly **24**. Bridge **42** also is useful for moving needle assembly **24** from the distal position to the proximal position. In particular, proximal portions of shield **44** can be engaged between a thumb and forefinger, while the index finger of the same hand engages the distal side of bridge **42** and pulls needle assembly **24** into the proximal position.

Needle assembly **24** also can be manipulated by rotating wings **38** and **40** toward one another and into substantially face-to-face engagement as shown in FIG. 3. Thus, the user can grip outwardly facing surfaces of wings **38** and **40** between a thumb and forefinger for manipulating needle assembly **24**.

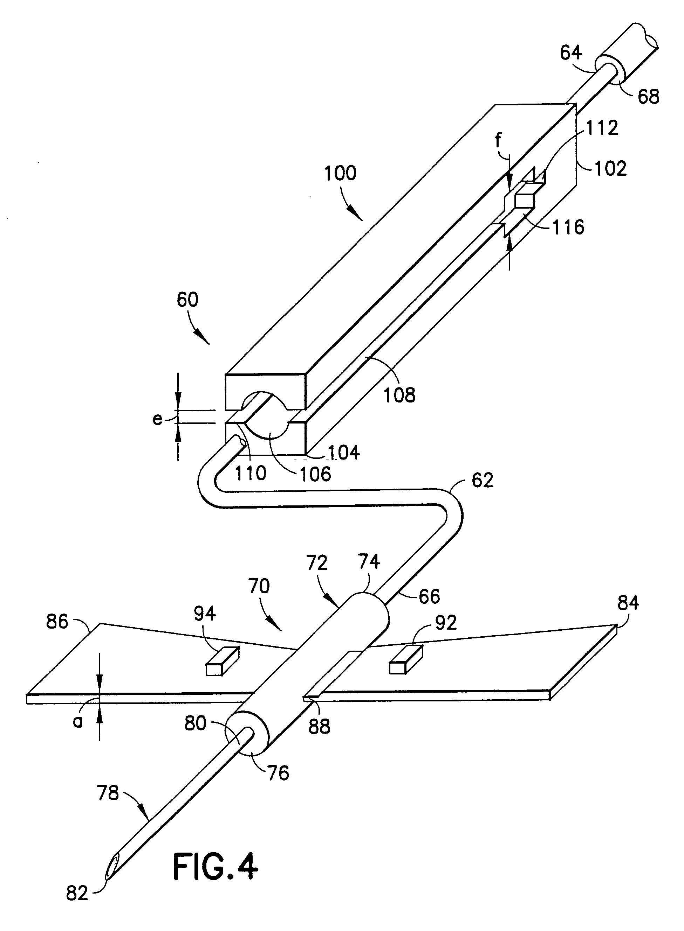

A second embodiment of the fluid collection or infusion set is identified by the numeral **60** in FIGS. 4-8. Fluid collection or infusion set **60** includes flexible tubing **62** with a proximal end **64** and a distal end **66**. Proximal end **64** is connected to a fitting **68** that may be identical to fitting **18** shown in FIG. 1. Distal end **66** of flexible tubing **62** is connected to a needle assembly **70** that is similar to needle assembly **24** shown in FIGS. 1-3. More particularly, needle assembly **70** includes a needle hub **72** with a proximal end **74**, a distal end **76** and a passage extending between the ends. Distal end **66** of plastic tubing **62** is connected securely to portions of the passage at proximal end **74** of needle hub **72**. Thus, the passage through needle hub **72** communicates with the passage through flexible tubing **62**. Needle assembly **70** further includes a needle cannula **78** having a proximal end **80** securely mounted in the passage of needle hub **72** adjacent distal end **76** of needle hub **72**. Needle cannula **78** further includes a distal end **82** and a lumen extending between the ends.

Needle assembly **70** also includes flexible wings **84** and **86** extending transversely from needle hub **72**. Wings **84** and **86** have a thickness "a" at most locations, but include thinned portions **88** and **90** adjacent needle hub **72**. Thinned portions **88** and **90** facilitate articulation of wings **84** and **86** about needle hub **72**. Wings **84** and **86** are formed from an elastomeric material and define a thickness at thin portions **88** and **90** that is selected to facilitate a slight resilient outward stretching of wings **84** and **86** away from needle hub **72**.

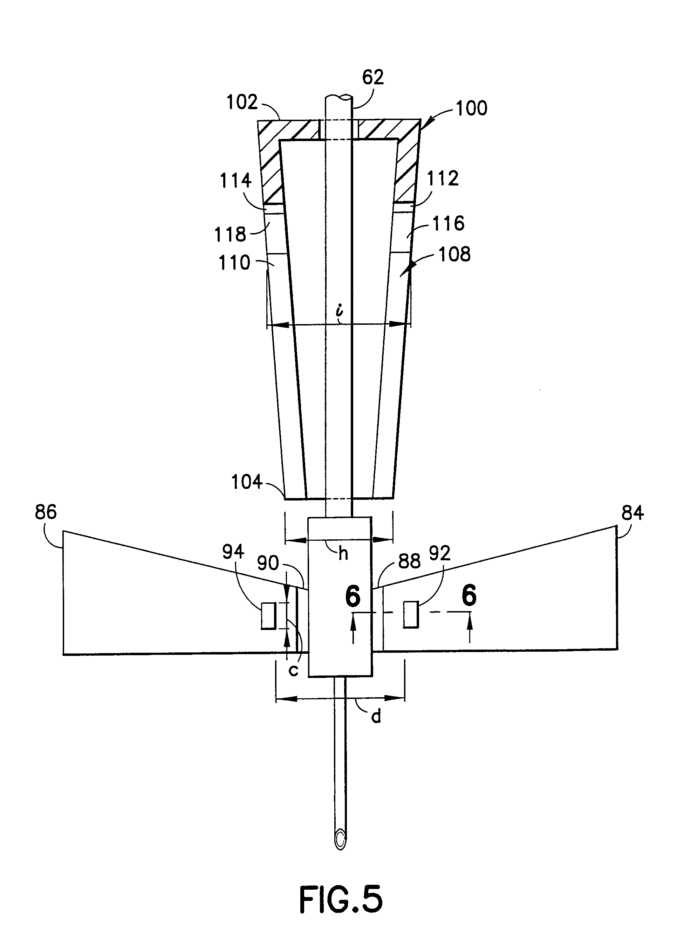

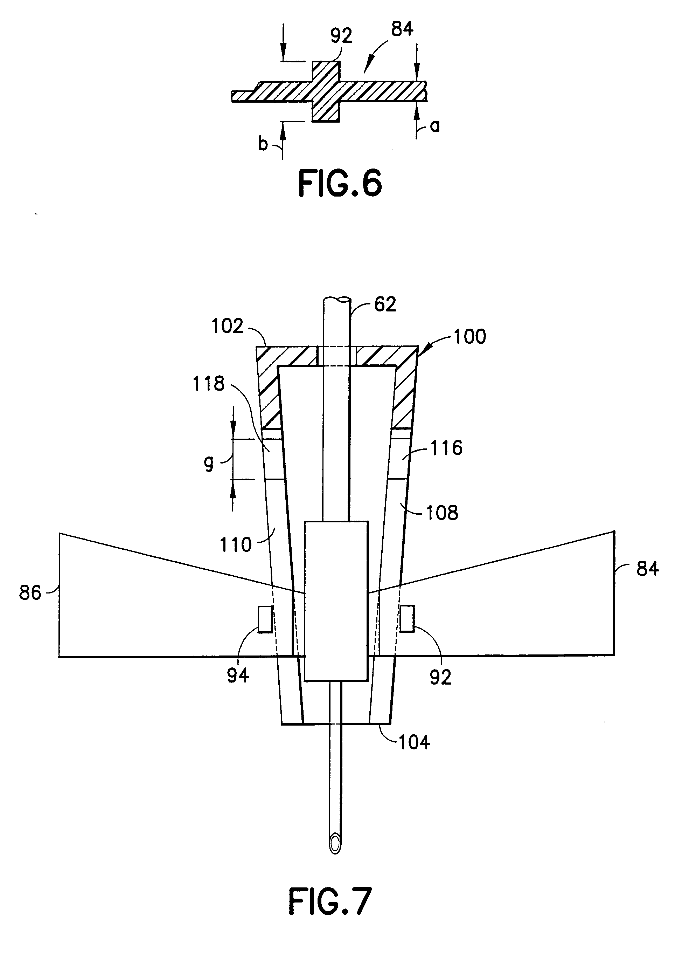

Wings **84** and **86** are formed with lugs **92** and **94** respectively projecting upwardly and downwardly from the top and bottom surfaces of wings **84** and **86** to define a thickness "b" as shown in FIG. 6 and a length "c" as shown in FIG. 5. Lugs **92** and **94** are spaced from one another by a distance "d" as shown in FIG. 5. Needle assembly **70** may further be provided with a bridge substantially identical to bridge **42** described and illustrated with respect to FIGS. 1-3. However, the bridge would have to be connected to wings **84** and **86** at locations outwardly from lugs **92** and **94**. Bridge **42** was an important feature of the first embodiment depicted in FIGS. 1-3. However, a bridge is not required for the second embodiment.

Fluid collection or infusion set **60** further comprises a shield **100**. Shield **100** is unitarily molded from a rigid plastic material and includes a proximal end **102**, a distal end **104** and a passage **106** extending between the ends. Shield **100** is further characterized by slots **108** and **110** that extend proximally from distal end **104** of shield **100**. Slots **108** and **110** are opposed to one another and substantially coplanar and extend entirely through peripheral walls of shield **100** from passage **106** to external locations. Additionally, slots **108** and **110** define a height "e" approximately equal to thickness "a" of wings **84** and **86** at locations near lugs **92** and **94**. Slots **108** and **110** have proximal ends **112** and **114** and recesses **116** and **118** immediately distally of proximal end **112** and **114**. Recesses define a height "f"' that exceeds the height "e" of distal portions of slots **108** and **110**. Additionally, heights 'f' of recesses **116** and **118** are slightly greater than thicknesses "b" of wings **84** and **86** at lugs **92** and **94**. Recesses **116** and **118** further define lengths "g" that are slightly greater than the lengths "c" of lugs **92** and **94**.

Shield **100** defines an external width "h" at distal end **104** which is less than distance "d" between lugs **92** and **94**. However, shield **100** flares to wider dimensions at locations closer to recesses **116** and **118**. In particular, shield **100** defines an external width "i" adjacent recesses **116** and **118** that is greater than distance "c" between lugs **92** and **94**.

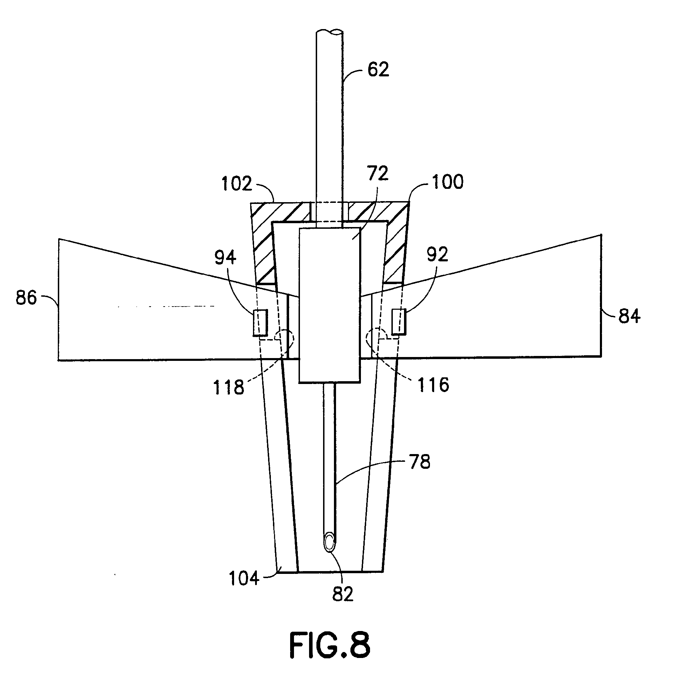

Fluid collection or infusion set **60** can be used substantially in a conventional manner with needle assembly **70** disposed distally of shield **100**, as shown in FIG. 4. Thus, wings **70** can be rotated toward one another in a conventional manner for one-handed manipulation of needle assembly **70**. After use, shield **100** is slid distally along tubing **62** and over proximal end **74** of needle hub **72**. Thus, wings **84** and **86** will enter slots **108** and **110**. Sufficient movement of shield **100** over needle assembly **70** will cause engagement between outer surfaces of shield **100** and lugs **92** and **94**, as shown in FIG. 7. This engagement effectively creates ramping forces that will cause a stretching of thinned regions **88** and **90** of wings **84** and **86** to permit further movement of shield **100** over needle assembly **70**. Sufficient distal advancement of shield **100** over needle assembly **70** will cause lugs **92** and **94** to align with recesses **116** and **118**. As noted above, recesses **116** and **118** define height "f" and length "g" dimensions that exceed the corresponding dimensions "b" and "c" for lugs **92** and **94**. Hence, wings **84** and **86** will resiliently return toward an unstretched condition and lugs **92** and **94** will enter recesses **116** and **118**. In this position, as shown in FIG. 8, distal end **82** of needle cannula **78** is safely disposed in shield **100**. The engagement of lugs **92** and 94 in recesses **116** and **118** will prevent a re-exposure of needle cannula **78**. In particular, re-exposure would require an extremely complex stretching of wings **84** and **86** away from one another and simultaneous proximal movement of shield relative to needle assembly **70**. However, flexible plastic tubing **62** is not structurally conducive to an accommodation of the forces that would be required to reexpose needle cannula **78**.

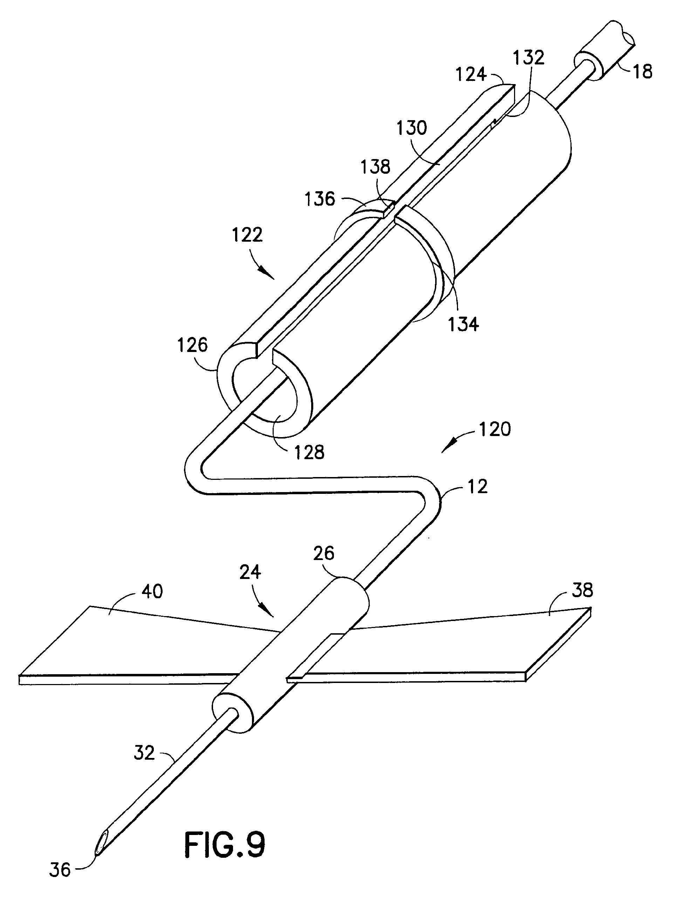

A third embodiment of the fluid collection or infusion set in accordance with the subject invention is identified generally by the numeral **120** in FIGS. 9-10. The fluid collection or infusion set **120** includes flexible plastic tubing **12**, a fitting **18** and a needle assembly **24**, all of which are substantially identical to comparably numbered elements in FIG. 1. However, fluid collection or infusion set **120** includes a shield **122** that is substantially different from the shield described and illustrated above. More particularly, shield **122** is a substantially cylindrical tube with a proximal end **124**, a distal end **126** and a passage **128** extending between the ends. Passage **128** is dimensioned to slidably accommodate hub **26** of needle assembly **24**. Shield **122** further includes a longitudinal slot 130 that extends from distal end **126** toward proximal end **124**. Slot **130** is dimensioned to accommodate sliding movement of wings **38** and **40** when the wings are folded into face-to-face engagement with one another. Thus, needle assembly **24** can be slid in a distal-to-proximal direction within shield **122** with folded wings **38** and **40** slidably moving through slot **130**. In the illustrated embodiment, slot **130** extends entirely to proximal end **124** of shield **122**. Proximal end **124** is characterized further by an inwardly extending flange **132** that limits proximal movement of needle assembly **24** in shield **122**. In other embodiments, however, slot **130** may terminate short of proximal end **124**, and hence the proximal end of slot **130** will limit proximal movement of needle assembly **24** relative to shield **122**.

Shield **122** further includes an annular groove **134** at an outer circumferential position thereon. A split annular clip **136** is rotatably mounted in groove **134**. Clip **136** includes a slit **138** defining a width approximately equal to the width of slot **130**. Clip **136** initially is in a rotatable position on shield **122** such that slit **138** of clip **136** aligns with slot **130** in shield **122**. However, clip **136** can be rotated into a position where slit **138** and longitudinal slit **130** are misaligned.

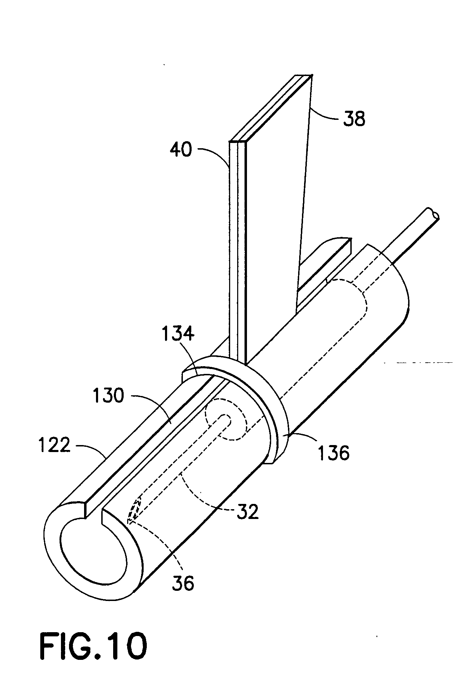

Fluid collection set **120** is used substantially in a conventional manner with needle assembly **24** disposed distally of shield **22**, as shown in FIG. 9. After use, wings **38** and **40** of needle assembly **24** are folded into face-to-face relationship with one another and shield **122** is slid distally relative to needle assembly **24**. Folded wings **38** and **40** move longitudinally through slot **130** of shield **122**. When needle assembly **24** reaches the extreme proximal position in shield **122**, distal end **36** of needle cannula **32** is safely disposed within shield **122**. At this time, wings **38** and **40** are disposed proximally of clip **136**. Clip **136** then is rotated in annular groove **134** of shield **122** from the FIG. 9 position, where slit **138** aligns with slot **130** to the position shown in FIG. 10 where clip **136** prevents a return distal movement of needle assembly **24** relative to shield **122**. Clip **136** and groove **134** may be formed with locking structures to prevent or substantially complicate a return rotational movement that could permit a re-exposure of needle cannula **32**. For example, groove **134** may be formed with a locking recess, and shield **136** may be formed with a locking projection that engages in the locking recess.

## Claims

1. A fluid collection set comprising a needle assembly having a needle hub, a needle cannula projecting from said hub, first and second flexible wings projecting transversely from said hub and being foldable into substantially face-to-face relationship with one another, a shield slidably movable relative to said needle assembly from a proximal position where said needle cannula is exposed to a distal position where said needle cannula is shielded, said shield including at least one longitudinal slot for slidably receiving said wings, said shield further comprising at least one lock for locking said needle assembly in said proximal position relative to said shield.

2. A fluid collection set according to claim 1, further comprising: a bridge connecting said first wing to said second wing at locations spaced from said needle hub, such that said bridge defines a finger actuation region for facilitating manipulation of said needle assembly.

3. A fluid collection set according to claim 2, wherein said shield includes opposite proximal and distal ends, said at least one slot comprising first and second slots dimensioned for slidably receiving said first and second wings respectively, such that said needle hub is slidably disposed in said shield and such that said wings project respectively through said first and second slots, said bridge being disposed externally of said shield.

4. A fluid collection according to any of claims 1 to 3, further comprising a length of flexible tubing connected to said needle hub and a fitting connected to said end of said flexible tubing remote from said needle hub.

5. A fluid collection set according to any of claims 1-4, wherein at least portions of said wings adjacent said needle hub are resiliently stretchable, said wings each being formed with a lug projecting therefrom, said shield being tapered from a minor width adjacent said distal end to a major width further from said distal end, said major width being greater than said distance between said lugs on said wings, said slots including recesses at proximal ends of said slots, said recesses being dimensioned for receiving said lugs, whereby said wings stretch as said major width of said shield approaches said lugs, and whereby said wings resiliently return to an unstretched condition with said lugs in said recesses when said recesses and said lugs align.

6. A fluid collection set according to claim 1, wherein said longitudinal slot is dimensioned for receiving said wings when said wings are folded into said face-to-face relationship with one another, a split clip ring having a slit formed therein, said split clip ring being mounted on said shield and being rotatable from a first position where said slit in said split clip ring aligns with said slot to a second position where said slit is offset from said slot, said split clip ring being disposed at a longitudinal position along said shield to be distally of said wings when said shield is in said distal position on said needle assembly, whereby rotation of said split clip ring when said shield is in said distal position on said needle assembly prevents re-exposure of said needle cannula.

7. A fluid collection set according to claim 6, further comprising locking means for securing said split clip ring in said position where said slit in said split clip ring is offset rotationally from said slot in said shield.

8. A fluid collection set according to claim 6 or 7, wherein said shield includes means for preventing said shield from moving distally beyond said needle assembly.

9. A fluid collection set according to claim 8, wherein the means comprises a flange extending inwardly at said proximal end of said shield.

10. A fluid collection set according to any of claims 6-9, wherein said shield includes an annular groove, said split clip ring being rotatably engaged in said annular groove.
